# EUROPÄISCHE PATENTSCHRIFT

(11) **EP 3 110 495 B1**
(45) Veröffentlichungstag und Bekanntmachung des Hinweises auf die Patenterteilung: **10.04.2019**
(21) Anmeldenummer: 15706792.7
(22) Anmeldetag: 24.02.2015
(51) Int. Cl.: A61M 39/16, A61M 39/10, F16B 39/30

(54) **SCHRAUBVERBINDER FÜR MEDIZINISCHE SCHLAUCHSYSTEME UND MEDIZINISCHES SCHLAUCHSYSTEM MIT SCHRAUBVERBINDER**
SCREW CONNECTOR FOR MEDICAL TUBE SYSTEMS, AND MEDICAL TUBE SYSTEMS HAVING SCREW CONNECTORS
RACCORD FILETÉ POUR SYSTÈMES DE TUYAUX MÉDICAUX ET SYSTÈME DE TUYAUX MÉDICAUX POURVU D'UN RACCORD FILETÉ

(30) Priorität: 27.02.2014 DE 102014002650
(43) Veröffentlichungstag der Anmeldung: 04.01.2017
(73) Patentinhaber: Fresenius Medical Care Deutschland GmbH, 61352 Bad Homburg (DE)
(72) Erfinder: LAUER, Martin, 66606 St. Wendel (DE)
(74) Vertreter: Rau, Jenspeter
(86) Internationale Anmeldenummer: PCT/EP2015/053841
(87) Internationale Veröffentlichungsnummer: WO 2015/128325

(56) Entgegenhaltungen:
- DE-C1- 3 515 665
- DE-U1-202009 013 409
- US-A- 3 952 899
- US-A- 5 096 083

## Beschreibung

### Technisches Gebiet

Die Erfindung betrifft Schraubverbinder für medizinische Schlauchsysteme und medizinische Schlauchsysteme mit Schraubverbinder sowie die Sterilisation solcher medizinischen Schlauchsysteme mittels Dampfsterilisierung und/oder Gassterilisierung. Es sind eine Vielzahl von Verbindern für medizinische Schlauchsysteme bekannt, insbesondere die als Luer-Lock Konnektoren genormten Schraubverbinder. Medizinische Schlauchsysteme kommen beispielsweise in der Infusionstechnik und in der extrakorporalen Blutbehandlung zum Einsatz und müssen für diese Anwendungen steril sein.

### Hintergrund

Zur Gewährleistung der Sterilität werden medizinische Schlauchsysteme für die Infusionstechnik und die extrakorporale Blutbehandlung in einer geeigneten geschlossenen Umhüllung verpackt und in dieser Verpackung sterilisiert. Die Sterilisierung kann beispielsweise als Dampfsterilisierung und/oder als Gassterilisierung erfolgen. Die vorgenannten Sterilisierverfahren erfordern, dass Gase und/oder Heißdampf zuverlässig an alle äußeren und inneren Oberflächen des medizinischen Schlauchsystems gelangen können.

Die Schrift DE 35 15 665 C1 offenbart einen Verschlussstopfen zum dichten Verschließen von Anschlüssen in der Medizintechnik, insbesondere von Luer-Lock-Anschlüssen. Seine beiden Verbindungsteile gestatten wahlweise ein loses Aufsetzen, bei dem ein Gasdurchtritt noch möglich ist, als auch das dichte Verschließen des Anschlusses. Die lose aufgesetzte Stellung und die Schließstellung sind durch einen Anschlag getrennt, der unter elastischer Verformung überwindbar ist. Dazu wird vorgeschlagen, den Anschlag mittels zwei innerer radialer Ringrippen vor dem Innengewinde des Verschlusstopfens und zwei äußeren Flügeln am komplementären Verbindungsteil auszuführen.

Ein Nachteil dieses bekannten Verschlussstopfens ist, dass die zwei inneren radialen Ringrippen nur durch eine zur Rotationsachse des Verschlussstopfens axial wirkende Druckkraft auf die Verbindungsteile einzeln und nacheinander überwunden werden können. Wenn die Druckkraft nicht exakt axial wirkt, kann es zum Verkanten kommen. Demzufolge muss der Anwender die Verbindungsteile sehr genau ausrichten, was dadurch erschwert wird, dass die Ringrippen unmittelbar vor dem Innengewinde des Verschlusstopfens angeordnet sind.

Beim Aufwenden einer nicht genau axial wirkenden Kraft oder einer zu kleinen axialen Kraft kann der Verschlusstopfen abfallen und im ungünstigsten Fall sogar auf den Boden fallen, so dass er nicht mehr steril ist. Daher ist der bekannte Verschlussstopfen unsicher und umständlich in der Handhabung.

Insgesamt führt das dazu, dass das Klinikpersonal mit fehleranfälligen und/oder umständlichen Prozeduren belastet wird.

Die Schrift WO 99/20376 A1 schlägt ein Verfahren zum Spülen eines Blutschlauchsatzes für die extrakorporale Blutbehandlung vor, wobei vorgeschlagen wird, den arteriellen Patientenkonnektor und den venösen Patientenkonnektor bereits während der Produktion des Blutschlauchsatzes mit einem Verbinder zu verbinden und in dieser verbundenen Form zu sterilisieren.

Die Schrift WO 96/40320 A1 schlägt ein weiteres Verfahren zum Spülen eines Blutschlauchsatzes für die extrakorporale Blutbehandlung vor, wobei vorgeschlagen wird, den arteriellen Patientenkonnektor und den venösen Patientenkonnektor beim Spülen des Blutschlauchsatzes über einen Y-Verbinder mit einem Ablauf zu verbinden. Dazu wird vorgeschlagen, dass der verpackte extrakorporale Blutschlauchsatz bereits den mit dem arteriellen Patientenkonnektor und dem venösen Patientenkonnektor verbundenen Y-Verbinder enthält.

Die Schrift WO 2010/121819 A1 der Anmelderin der vorliegenden Patentanmeldung offenbart eine Blutkassette mit einem Blutschlauchsatz für die extrakorporale Blutbehandlung mit einer arteriellen Patientenleitung und einer venösen Patientenleitung. Des Weiteren offenbart die Schrift WO 2010/121819 A1 auch ein Verfahren zum Spülen eines extrakorporalen Blutkreislaufs, wobei die Patientenkonnektoren der arteriellen Patientenleitung und der venösen Patientenleitung mittels eines T-Verbinders an dem Rinse-Port der Dialysemaschine angeschlossen wird. Auf den Inhalt der Schrift WO 2010/121819 A1 wird in der vorliegenden Patentanmeldung vollumfänglich Bezug genommen.

Es sind Kegelverbindungen mit einem 6 % (Luer) Kegel für Spritzen, Kanülen und bestimmte andere medizinische Geräte, insbesondere verriegelbare Kegelverbindungen bekannt, die auch als Luer-Lock-Verbindungen bezeichnet werden.

Die Schrift US 5 096 083 offenbart einen Transportbehälter für Gefahrstoffe mit einem Verschlussdeckel mit selbstsicherndem Gewinde um den sicheren Verschluss des Transportbehälters während des Transports zu gewährleisten.

Die Schrift US 3 952 899 offenbart einen Behälter mit Schraubdeckel, wobei das Gewinde selbstsichernd ausgeführt ist um den Behälter zuverlässig fluiddicht verschlossen zu halten.

Die Schrift DE 20 2009 013 409 U1 offenbart eine Verschlusskappe mit Gewinde und Berührschutz für deren Innenkonus für einen medizinischen Schlauchsatz.

Die Schrift DE 35 15 665 C1 offenbart eine Kappe zum Verschließen eines medizinischen Schlauchsatzes bei der mittels einer ringförmigen Schnappverbindung am inneren Umfang der Kappe eine halboffene Stellung der Schraubverbindung bereitgestellt wird.

### Zusammenfassung der Erfindung

Es ist daher eine Aufgabe der vorliegenden Erfindung mindestens einen der genannten Nachteile zu überwinden und einen verbesserten Schraubverbinder für medizinische Schlauchsysteme bereitzustellen.

Diese Aufgabe wird gelöst mit den Merkmalen der unabhängigen Patentansprüche 1, 11 und 16. Vorteilhafte Ausführungsformen sind in den Unteransprüchen angegeben.

Die vorliegende Erfindung stellt einen Schraubverbinder für ein medizinisches Schlauchsystem bereit mit einem ersten Verbindungselement mit einem ersten Lumen und einem Außengewinde und einem zweiten Verbindungselement mit einer elastisch verformbaren Mutter mit einem Innengewinde, wobei das Innengewinde und das Außengewinde konfiguriert sind um miteinander verschraubt zu werden, wobei das Außengewinde eine erste Ausformung aufweist und die Mutter eine zweite Ausformung aufweist, wobei die zweite Ausformung eine Aussparung im Gewindegrund der Mutter ist. Das Innengewinde und das Außengewinde sind konfiguriert sind zum gegenseitigen Verschrauben und die Mutter ist konfiguriert zur elastischen Verformung durch die erste Ausformung in einem vorgegebenen ersten Drehwinkelbereich, ohne dass die erste Ausformung in die Aussparung im Gewindegrund der Mutter einrastet. Das Außengewinde und die erste Ausformung und die Aussparung im Gewindegrund der Mutter sind konfiguriert sind zum Einrasten der ersten Ausformung in die Aussparung im Gewindegrund der Mutter, wenn das Innengewinde und das Außengewinde in einem vorgegebenen zweiten Drehwinkelbereich verschraubt sind.

Der Begriff des Schraubverbinders ist im Zusammenhang mit der vorliegenden Erfindung nicht eingeschränkt auf eine bestimmte Ausführungsform der flüssigkeitsdichten Verbindung eines ersten Lumens mit einem zweiten Lumen. Die flüssigkeitsdichte Verbindung, insbesondere durch Dichtungsflächen, kann unabhängig von der Verschraubung sein und/oder zusätzlich zu der Verschraubung vorliegen. Die Verschraubung kann insbesondere eine zusätzliche Sicherung und/oder Verriegelung einer flüssigkeitsdichten Verbindung eines ersten Lumens mit einem zweiten Lumen gegen unbeabsichtigtes Lösen umfassen.

In einer Ausführungsform sind das erste Verbindungselement und das zweite Verbindungselement jeweils als komplementäre Hälfte eines gemeinsamen Luer-Lock Verbinders ausgeführt. Das Innengewinde und das Außengewinde sind jeweils als komplementäre Gewinde des Luer-Lock Verbinders ausgeführt und dienen als zusätzliche Sicherung des Luer-Konus gegen unbeabsichtigtes Lösen. Die komplementären Gewinde eines Luer-Lock Verbinders sind zumindest hinsichtlich Gewindeform, Gewindedurchmesser, Gewindesteigung und Gewindelänge kompatibel ausgeführt und so zum gegenseitigen Verschrauben konfiguriert und vorgesehen.

Die Gewindemutter des Schraubverbinders ist vorzugsweise kompatibel mit den komplementären Hälften standardisierter Verbinder, weil die zweite Ausformung im Gewindegrund des Innengewindes der Gewindemutter gegenüber den komplementären Hälften standardisierter Verbinder nicht störend wirkt, so dass beispielsweise das Verschrauben von standardisierten Luer-Konnektoren sowie Dialysenadeln, Kanülen, Kathetern oder Ports mit der erfindungsgemäßen Gewindemutter nicht beeinträchtigt wird.

Das Außengewinde weist bevorzugt eine erste Ausformung auf, die eine lokale Erhebung auf der Gewindeflanke des Außengewindes, insbesondere überwiegend oder ausschließlich auf dem Außendurchmesser des Außengewindes ist. Dadurch ergibt sich im Bereich der Erhebung lokal ein vergrößerter Durchmesser des Außengewindes.

In einer Ausführungsform ist die erste Ausformung auf der Gewindeflanke des Außengewindes eine Nase oder eine Nocke. Der Durchmesser des Außengewindes ist dann nur im Bereich der Nocke oder Nase vergrößert.

In einer anderen Ausführungsform umfasst die erste Ausformung mehrere voneinander beabstandete lokale Erhebungen auf der Gewindeflanke des Außengewindes. Der Durchmesser des Außengewindes ist dann jeweils im Bereich jeder der lokalen Erhebungen vergrößert.

In einer weiteren Ausführungsform umfasst die erste Ausformung mehrere Nasen oder Nocken auf der Gewindeflanke des Außengewindes. Der Durchmesser des Außengewindes ist dann jeweils im Bereich jeder der Nocken oder Nasen vergrößert.

Die Mutter weist eine zweite Ausformung auf, die eine Aussparung im Gewindegrund der Mutter ist.

In einer Ausführungsform ist die zweite Ausformung eine Vertiefung im Gewindegrund der Mutter. Daher ist der Durchmesser des Gewindegrunds des Innengewindes im Bereich der Vertiefung größer als in anderen Abschnitten der Mutter.

In einer anderen Ausführungsform umfasst die zweite Ausformung zumindest eine Durchgangsöffnung im Gewindegrund der Mutter zwischen dem Inneren der Mutter und einer äußeren Umgebung der Mutter. Eine solche Ausführungsform ist besonders vorteilhaft um durch Spritzgießen hergestellt zu werden, weil sich das Kernwerkzeug für die Durchgangsöffnung problemlos entformen lässt.

In einer weiteren Ausführungsform umfasst die zweite Ausformung zumindest eine radiale Nut in einer axialen Stirnseite der Mutter, wobei die radiale Nut in Richtung der axialen Stirnseite der Mutter offen ist. Eine solche Ausführungsform ist besonders vorteilhaft um durch Spritzgießen hergestellt zu werden, weil sich das Kernwerkzeug für die radiale Nut problemlos entformen lässt.

Im Einklang mit der Lehre der vorliegenden Erfindung ist die Mutter konfiguriert zur elastischen Verformung durch die erste Ausformung in einem vorgegebenen ersten Drehwinkelbereich, in dem die erste Ausformung nicht in die Aussparung im Gewindegrund der Mutter einrastet. Dazu kann der Durchmesser der Mutter beim Verdrehen in einem ersten Drehwinkelbereich elastisch aufgeweitet werden, wenn eine Nocke oder Nase auf der Gewindeflanke des Außengewindes im Inneren der Mutter gegen den Gewindegrund der Mutter drückt und/oder auf dem Gewindegrund entlanggleitet. Anders ausgedrückt trifft die erste Ausformung in dem vorgegebenen ersten Drehwinkelbereich auf keine zweite Ausformung, so dass die erste Ausformung in dem vorgegebenen ersten Drehwinkelbereich in keine zweite Ausformung einrasten kann. Das Innengewinde und das Außengewinde und die erste Ausformung und die Aussparung im Gewindegrund der Mutter sind des Weiteren konfiguriert zum Einrasten der ersten Ausformung in die Aussparung im Gewindegrund der Mutter, wenn das Innengewinde und das Außengewinde in einem vorgegebenen zweiten Drehwinkelbereich verschraubt sind. Beispielsweise rastet eine Nocke oder Nase auf der Gewindeflanke des Außengewindes in die Aussparung im Gewindegrund der Mutter ein, wenn das Innengewinde und das Außengewinde in einem vorgegebenen zweiten Drehwinkelbereich verschraubt sind. Beim Einrasten in dem zweiten Drehwinkelbereich bildet sich die elastische Verformung der Mutter zurück, weil keine Nocke oder Nase auf der Gewindeflanke des Außengewindes im Inneren der Mutter gegen den Gewindegrund der Mutter drückt. Der zweite Drehwinkelbereich kann durch die Form und die Anordnung der ersten und/oder der zweiten Ausformung festgelegt sein.

In einer Ausführungsform weist die Mutter an ihrem äußeren Umfang zumindest eine in axialer Richtung der Mutter angeordnete Versteifungsrippe auf, wobei sich die mindestens eine Durchgangsöffnung zumindest teilweise entlang der Versteifungsrippe erstreckt.

In einer weiteren Ausführungsform sind am äußeren Umfang der Mutter in axialer Richtung der Mutter eine Mehrzahl Versteifungsrippen angeordnet, wobei sich die mindestens eine Durchgangsöffnung zumindest teilweise entlang und zwischen zwei Versteifungsrippen erstreckt.

In einer weiteren Ausführungsform weist das zweite Verbindungselement eine Abdichtkappe mit einer Abdichtstruktur auf oder ist eine Abdichtkappe mit einer Abdichtstruktur. Eine solche Ausführungsform kann beispielsweise vorliegen, wenn das zweite Verbindungselement als Berührschutzkappe und/oder Verschlusskappe konfiguriert ist. Die Abdichtstruktur kann zum gasdichten und/oder flüssigkeitsdichten Abdichten mit einem ersten Verbindungselement konfiguriert sein.

In einer weiteren Ausführungsform ist das erste Lumen als weibliche Hälfte eines Luer-Konnektors oder Luer-Lock Konnektors ausgebildet und die Abdichtstruktur als männliche Hälfte des Luer-Konnektors ausgebildet.

In einer weiteren Ausführungsform weist das zweite Verbindungselement ein als zweites Lumen bezeichnetes Lumen auf und die Mutter ist als Überwurfmutter konfiguriert. Die Überwurfmutter ist drehbar am zweiten Verbindungselement gelagert, so dass die Überwurfmutter mit einem ersten Verbindungselement verschraubt werden kann, ohne dass sich das zweite Verbindungselement verdreht. Eine solche Ausführungsform ist besonders vorteilhaft, wenn der erfindungsgemäße Schraubverbinder zum Verbinden von zwei Schlauchleitungsabschnitten vorgesehen ist, die sich beim Verschrauben des Schraubverbinders nicht verdrehen sollen.

In einer weiteren Ausführungsform ist das erste Lumen als weibliche Hälfte eines Luer-Konnektors ausgebildet und das zweite Lumen als männliche Hälfte des Luer-Konnektors ausgebildet.

Ein Vorteil der vorliegenden Erfindung ist, dass ein Schraubverbinder für medizinische Schlauchsysteme bereitgestellt wird, der eine halboffene Sterilisierstellung zulässt und zugleich ergonomisch und sicher in der Handhabung ist. Gleichzeitig ist die halboffene Sterilisierstellung verlässlich und sicher.

Unter einer halboffenen Sterilisierstellung kann in manchen Ausführungsformen verstanden werden, dass die beiden komplementären Hälften eines Luer-Lock-Verbinders nicht dichtend verbunden sind sondern dass zwischen den komplementären Konusflächen des Luer-Lock-Verbinders ein definierter Spalt eingestellt ist. Dadurch kann beim Dampf- und/oder Gassterilisieren der Dampf- und/oder das Gas durch den Spalt auch zwischen die komplementären Hälften und in das jeweilige Innere der komplementären Hälften des Luer-Lock-Verbinders gelangen. Der Begriff "halboffen" wird verwendet um deutlich zu machen, dass die beiden komplementären Hälften des Luer-Lock-Verbinders jeweils zumindest teilweise durch die jeweils komplementäre Hälfte des Luer-Lock-Verbinders abgedeckt sind. Das hat den Vorteil, dass für beide komplementären Hälften des Luer-Verbinders trotz des definierten Spalts ein Berührschutz gewährleistet ist, ohne dass dafür zusätzliche Berührschutzkappen erforderlich sind.

In manchen Ausführungsformen kann die halboffene Sterilisierstellung den Vorteil aufweisen, dass die beide komplementären Hälften des Luer-Lock-Verbinders bereits vormontiert sind und für die Anwendung nur noch dichtend verbunden und durch Zuschrauben gesichert werden müssen. Solche Ausführungsformen sind besonders anwenderfreundlich, weil dem Anwender der Arbeitsgang des Konnektierens des Luer-Verbinders erleichtert und/oder zumindest teilweise abgenommen wird.

In einer weiteren Ausführungsform ist der Schraubverbinder konfiguriert um in einem weiteren Drehwinkelbereich, beispielsweise einem dritten Drehwinkelbereich, gas- und/oder flüssigkeitsdicht verschraubt zu werden. Dabei sind das erste Verbindungselement und das zweite Verbindungselement jeweils konfiguriert um beim Verschrauben des Innengewindes mit dem Außengewinde in dem zweiten Drehwinkelbereich zwischen einer äußeren Umgebung des Schraubverbinders und einem zumindest durch das erste Lumen gebildeten Inneren des Schraubverbinders gasdurchlässig zu sein und außerdem sind das erste Verbindungselement und das zweite Verbindungselement jeweils konfiguriert um beim Verschrauben des Innengewindes mit dem Außengewinde in dem weiteren Drehwinkelbereich gas- und/oder flüssigkeitsdicht zwischen der äußeren Umgebung des Schraubverbinders und dem Inneren des Schraubverbinders abzudichten. Zum Verschrauben in dem weiteren Drehwinkelbereich ist zunächst ein Überschreiten des zweiten Drehwinkelbereichs erforderlich. Anders ausgedrückt wird der Schraubverbinder über den zweiten Drehwinkelbereich hinaus weiter zugedreht. Dabei rastet zunächst die erste Ausformung aus der zweiten Ausformung aus. Für das Ausrasten der ersten Ausformung aus der zweiten Ausformung ist ein Drehmoment an der Mutter erforderlich. Die gas- und/oder flüssigkeitsdichte Stellung wird erst durch vollständiges Zudrehen der Schraubverbindung erreicht.

Der zweite Winkelbereich kann in manchen Ausführungsformen im Wesentlichen durch die Breite der ersten Ausformung und die Breite der zweiten Ausformung in Drehrichtung des Gewindes festgelegt sein. Dabei kann die zweite Ausformung gegenüber der ersten Ausformung ein Übermaß aufweisen, so dass sich ein Spiel ergibt. Das Spiel kann das Einrasten der ersten Ausformung in die zweite Ausformung erleichtern.

In einer weiteren Ausführungsform ist der erfindungsgemäße Schraubverbinder Teil eines medizinischen Schlauchsystems. Der Begriff "medizinisches Schlauchsystem" umfasst in der vorliegenden Erfindung alle medizinischen Produkte, die zumindest einen Schlauchabschnitt aufweisen und insbesondere für die extrakorporale Blutbehandlung, insbesondere für die Hämodialyse, Hämofiltration oder Hämodiafiltration, oder für die Peritonealdialyse oder für die Infusion von medizinischen Lösungen in den Patienten konfiguriert und/oder vorgesehen sind.

Eine Ausführungsform des erfindungsgemäßen medizinischen Schlauchsystems ist ein Blutschlauchsatz für die extrakorporale Blutbehandlung mit einem arteriellen Patientenschlauch und einem venösen Patientenschlauch, wobei zumindest das patientenseitige Ende des venösen Patientenschlauchs und/oder das patientenseitige Ende des arteriellen Patientenschlauchs als das erste Verbindungselement oder das zweite Verbindungselement des erfindungsgemäßen Schraubverbinders ausgebildet sind.

Es ist ein weiterer Vorteil der vorliegenden Erfindung, dass ein vor-konnektierter Blutschlauchschlauchsatz für die extrakorporale Blutbehandlung mit einem Drei-Wege-Verbinder bereitgestellt wird, der zum Sterilisieren des Blutschlauchsatzes eine halboffene Sterilisierstellung der an dem Drei-Wege-Verbinder vor-konnektierten Luer-Lock-Verbindungen des arteriellen und des venösen Patientenkonnektors ermöglicht und zugleich ergonomisch und sicher in der Handhabung ist. Dabei ergibt sich der zusätzliche Vorteil, dass zwei Verschlusskappen zum Verschließen des arteriellen und des venösen Patientenkonnektors eingespart werden.

Der Erfinder der vorliegenden Erfindung hat erkannt, dass die komplementären Hälften eines vom Standard abweichenden Schraubverbinders eines medizinischen Schlauchsatzes beim Einsatz in der Klinik nicht nur eingeschränkt, sondern möglichst flexibel, insbesondere kompatibel oder sogar kreuzkompatibel einsetzbar sein sollten.

In einer bestimmten Ausführungsform des Schraubverbinders ist sowohl das erste Verbindungselement als auch das zweite Verbindungselement jeweils mit einem Gegenstück eines genormten Luer-Lock Verbinders zum flüssigkeitsdichten Verschrauben kompatibel, weil weder die erste Ausformung noch die zweite Ausformung dabei störend wirken. Es kann somit in der Klinik eine Hälfte des erfindungsgemäßen Schraubverbinders mit einem Gegenstück eines standardisierten Luer-Lock Verbinders verbunden werden. Die Verwendung des erfindungsgemäßen Schraubverbinders beim Sterilisieren eines medizinischen Schlauchsatzes schränkt daher die Verwendung des medizinischen Schlauchsatzes in der Klinik nicht ein. Daher ist sowohl das erste Verbindungselement als auch das zweite Verbindungselement jeweils kreuzkompatibel und daher flexibel im Klinikbetrieb einsetzbar. Als ein Beispiel werden Dialysenadeln angeführt, die einen kurzen Schlauchabschnitt mit einer Hälfte eines genormten Luer-Lock Verbinders aufweisen. Diese sind ohne Einschränkung mit dem komplementären Gegenstück eines erfindungsgemäßen Schraubverbinders verbindbar und zum Verbinden vorgesehen. Daher können die Hälften des erfindungsgemäßen Schraubverbinders an der venösen Patientenleitung und/oder der arteriellen Patientenleitung ohne Einschränkung an der komplementären Hälfte eines genormten Luer-Lock Verbinders einer arteriellen und/oder venösen Dialysenadel verbunden werden.

Somit sind die Hälften des erfindungsgemäßen Schraubverbinders jeweils kreuzkompatibel mit den komplementären Hälften eines bestimmten genormten Schraubverbinders.

Der Schraubverbinder ist vorzugsweise unempfindlich gegenüber axialem Druck oder Zug am ersten und/oder zweiten Verbindungselement wenn das Innengewinde mit dem Außengewinde verschraubt ist. Der Schraubverbinder ist dann robust gegenüber dem unbeabsichtigten Diskonnektieren aufgrund von Zug- und/oder Biegebeanspruchungen der Verbindungsteile, wie diese bei Verpackung, Lagerung und Transport auftreten können, weil die Gewindeflanken die Zug- und Biegekräfte aufnehmen. Das Lösen ist nur durch eine gezielte Drehbewegung über einen Widerstand hinaus möglich.

Die elastische Verformung der Mutter oder Überwurfmutter beim Verschrauben in dem ersten Drehwinkelbereich beträgt beispielsweise weniger als fünf zehntel Millimeter und mehr als fünf hundertstel Millimeter. Der Außendurchmesser des Außengewindes des ersten Verbindungselements weist im Bereich der ersten Ausformung gegenüber dem Durchmesser des Gewindegrunds des Innengewindes des zweiten Verbindungselements, beispielsweise ausgeführt als Mutter oder Überwurfmutter, bevorzugt ein Übermaß von vorzugsweise 0,1 mm bis 0,3 mm auf. Anders ausgedrückt ergibt sich zwischen einer ersten Ausformung und dem Durchmesser des Gewindegrunds des Innengewindes des zweiten Verbindungselements ein radiales Übermaß von vorzugsweise 0,05 mm bis 0,15 mm. Dieses Übermaß führt beim gegenseitigen Verschrauben des Innengewindes mit dem Außengewindes in dem vorgegebenen ersten Drehwinkelbereich, in dem die erste Ausformung nicht in die zweite Ausformung im Gewindegrund der Mutter einrastet, zur elastischen Verformung der Mutter oder Überwurfmutter durch die erste Ausformung.

Der Schraubverbinder kann in einer Ausführungsform für nur ein einmaliges Einrasten der ersten Ausformung in die zweite Ausformung konfiguriert sein. Ein mehrmaliges Einrasten ist in solchen Ausführungsformen nicht vorgesehen, weil die erste Ausformung und/oder die zweite Ausformung beim Einrasten und Verschrauben über die Einrastung hinaus verschleißen. Solche Ausführungsformen sind aufgrund der geringen Anforderungen an Haltbarkeit der ersten Ausformung und/oder der zweite Ausformung besonders kostengünstig.

In einer Ausführungsform des Schraubverbinders ist eine Führungshilfe beziehungsweise Führungsstrecke vorgesehen, die die Handhabung durch den Anwender in der Klinik erleichtert und außerdem eine Montage durch Roboter ermöglicht. Die Führungshilfe kann in manchen Ausführungsformen durch das Gewinde selbst realisiert sein.

Ein Vorteil der vorliegenden Erfindung ist, dass ein Schraubverbinder bereitgestellt wird, der ergonomisch und sicher in der Handhabung ist.

Die Hälften des Schraubverbinders können so beim manuellen Zuschrauben und Abschrauben in der Klinik sicher geführt und gehalten werden, was besonders vorteilhaft ist, wenn der Schraubverbinder kleinteilig ist. Der Außendurchmesser der Mutter des erfindungsgemäßen Schraubverbinders kann beispielsweise 6 Millimeter bis 15 Millimeter betragen.

Bei größeren Schraubverbindern kann der Außendurchmesser 15 Millimeter bis 25 Millimeter betragen.

Typischerweise erfolgt das manuelle Auf- und/oder Zuschrauben des erfindungs-gemäßen Schraubverbinders durch Verdrehen der Mutter oder der Überwurfmutter des zweiten Verbindungselements zwischen den Fingerkuppen von Daumen und Zeigefinger der einen Hand des Anwenders während das erste Verbindungselement von den Fingern der anderen Hand festgehalten wird. Beim Auf- und/oder Zuschrauben des erfindungsgemäßen Schraubverbinders spürt der Anwender zum einen von Anfang an, dass das erste Verbindungselement gegenüber dem zweiten Verbindungselement um einen Drehwinkel verdreht und vom Gewinde sicher geführt wird. Zum anderen spürt der Anwender in Abhängigkeit vom Drehwinkel einen veränderten Widerstand beim Verdrehen, beispielsweise erstens wenn die Mutter oder Überwurfmutter unter deren elastischer Verformung durch die erste Ausformung in dem vorgegebenen ersten Drehwinkelbereich verdreht wird, ohne dass die erste Ausformung in die Aussparung im Gewindegrund der Mutter einrastet und zweitens wenn die erste Ausformung in die Aussparung im Gewindegrund der Mutter einrastet, wenn das Innengewinde und das Außengewinde in einem vorgegebenen zweiten Drehwinkelbereich verschraubt sind. Des Weiteren spürt der Anwender einen veränderten Widerstand beim Verdrehen wenn das Innengewinde und das Außengewinde über den vorgegebenen zweiten Drehwinkelbereich hinaus verschraubt und flüssigkeitsdicht zugeschraubt werden. Der Anwender kann diese veränderlichen Widerstände jeweils auch beim Aufschrauben bis zum Trennen des ersten Verbindungselements von dem zweiten Verbindungselement spüren. Wenn der Schraubverbinder mehrere Einraststellungen aufweist, dann kann der Anwender die veränderlichen Widerstände bei jeder Einraststellung spüren.

Der Schraubverbinder ermöglicht somit durch den drehwinkelabhängigen Drehwiderstand ein taktiles Feedback bei der manuellen Handhabung des erfindungsgemäßen Schraubverbinders, das heißt die Vermittlung des Gefühls der Ertastbarkeit des Handhabungsfortschritts mit den Fingern. Vorteilhaft ist dabei der durch das Gewinde verlängerte Weg zwischen dem nicht verrasteten Zustand und dem verrasteten Zustand und umgekehrt.

Der erfindungsgemäße Schraubverbinder ist somit vorteilhaft auch ergonomisch und sicher in der Handhabung.

Die Mutter oder die Überwurfmutter des zweiten Verbindungselements kann durch die Auswahl eines geeigneten elastischen Werkstoffs und die Formgebung zur elastischen Verformung durch die erste Ausformung des ersten Verbindungselements konfiguriert sein.

Als Werkstoff für das erste und das zweite Verbindungselement eignen sich beispielsweise Kunststoffe.

Als Werkstoff für das erste und das zweite Verbindungselement eignet sich in manchen Ausführungsformen insbesondere PP (Polypropylen), PC (Polycarbonat), PVC (Polyvinylchlorid) oder ABS (Acrylnitril-Butadien-Styrol).

### Kurzbeschreibung der Figuren

Im Folgenden wird ein Ausführungsbeispiel nach der vorliegenden Lehre unter Bezugnahme auf die Figuren näher erläutert. Anhand des in den

Figuren dargestellten Ausführungsbeispiels werden weitere Einzelheiten und Vorteile näher beschrieben. Die Bezugszeichen in den Figuren haben jeweils durchgängig in allen Figuren die gleiche Bedeutung.

Es zeigen:
**Fig. 1** eine Schnittdarstellung eines ersten Ausführungsbeispiels des erfindungsgemäßen Schraubverbinders
**Fig. 2a** eine erste Ansicht des ersten Verbindungselements gemäß dem ersten Ausführungsbeispiel aus Figur 1
**Fig. 2b** eine um 90° gedrehte zweite Ansicht des ersten Verbindungselements gemäß dem ersten Ausführungsbeispiel aus Figur 1
**Fig. 3** eine Ansicht eines ersten Ausführungsbeispiels des zweiten Verbindungselements gemäß dem ersten Ausführungsbeispiel des erfindungsgemäßen Schraubverbinders aus Figur 1
**Fig. 4** eine Ansicht eines zweiten Ausführungsbeispiels des zweiten Verbindungselements gemäß dem ersten Ausführungsbeispiel des erfindungsgemäßen Schraubverbinders aus Figur 1
**Fig. 5** eine Ansicht eines dritten Ausführungsbeispiels des zweiten Verbindungselements gemäß dem ersten Ausführungsbeispiel des erfindungsgemäßen Schraubverbinders aus Figur 1
**Fig. 6** eine Schnittdarstellung und eine Ansicht eines zweiten Ausführungsbeispiels des erfindungsgemäßen Schraubverbinders.

### Detaillierte Beschreibung der Figuren

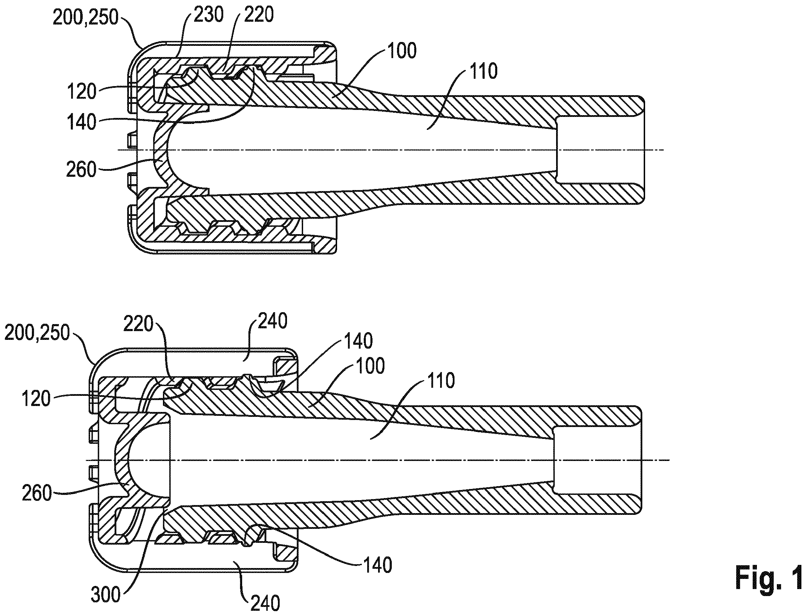

Die **Fig. 1** zeigt eine Schnittdarstellung eines ersten Ausführungsbeispiels eines Schraubverbinders in geschlossen verschraubter Stellung (obere Hälfte der Figur) und in halboffener Sterilisierstellung (untere Hälfte der Figur). Der Schraubverbinder weist ein erstes Verbindungselement 100, ausgeführt als weibliche Hälfte eines Luer-Konnektors, mit einem ersten Lumen 110 und einem Außengewinde 120 sowie ein zweites Verbindungselement 200 in Form einer Abdichtkappe 250, ausgeführt als männliche Hälfte des Luer-Konnektors mit einem Innengewinde 220 und einer Abdichtstruktur 260 auf. Das zweite Verbindungselement ist als Verschlusskappe oder Abschlusskappe in Form einer Mutter 230 ausgeführt. Das Außengewinde 120 weist zumindest eine erste Ausformung 140 auf. Die erste Ausformung ist als eine lokale Erhebung, beispielsweise als Nocke oder Nase, auf der Gewindeflanke des Außengewindes 120 angeordnet und ragt über den Gewindedurchmesser des Außengewindes hinaus. Das Innengewinde 220 weist eine zweite Ausformung 240 auf, wobei die zweite Ausformung eine Aussparung im Gewindegrund der Mutter 230 ist. Die Aussparung im Gewindegrund der Mutter ist in diesem Ausführungsbeispiel als Durchgangsöffnung zwischen dem Inneren der Mutter und einer äußeren Umgebung der Mutter ausgeführt.

Das Innengewinde 220 und das Außengewinde 120 sind konfiguriert zum gegenseitigen Verschrauben und die Mutter 230 ist konfiguriert zur elastischen Verformung durch die erste Ausformung in einem vorgegebenen ersten Drehwinkelbereich, ohne dass die erste Ausformung in die Aussparung im Gewindegrund der Mutter einrastet. Das Innengewinde 220 und das Außengewinde 120 sowie die erste Ausformung und die Aussparung im Gewindegrund der Mutter sind konfiguriert zum Einrasten der ersten Ausformung in die Aussparung im Gewindegrund der Mutter, wenn das Innengewinde und das Außengewinde in einem vorgegebenen zweiten Drehwinkelbereich verschraubt sind (untere Abbildung der Figur 1). Die Mutter 230 ist aus einem elastisch verformbaren Kunststoff gefertigt, beispielsweise PP (Polypropylen). Dies ermöglicht eine elastische Verformung der Mutter 230 durch die erste Ausformung in dem vorgegebenen ersten Drehwinkelbereich. Die obere Abbildung der Figur 1 zeigt den Schraubverbinder im gas- und/oder flüssigkeitsdicht verschraubten Zustand. Die Abdichtstruktur 260 liegt über deren gesamten Umfang dichtend an der Innenfläche des ersten Lumens 110 an und dichtet das erste Lumen 110 gegen eine äußere Umgebung des Schraubverbinders ab. Die untere Abbildung der Figur 1 zeigt den Schraubverbinder in der halboffenen Sterilisierstellung, bei der zwischen dem ersten Lumen 110 und der Abdichtstruktur 260 ein Spalt 300 verbleibt, der einen Gas- und Dampfdurchtritt ermöglicht. Somit können das Sterilisationsgas und/oder der Sterilisationsdampf beim Sterilisieren in das Lumen 110 eindringen und die Oberflächen sterilisieren.

Das erste Verbindungselement und das zweite Verbindungselement können vorzugsweise so konfiguriert sein, dass der Spalt zwischen den Konus-Dichtflächen der komplementären Hälften des Luer-Lock-Verbinders in der halboffenen Sterilisierstellung eine Strömungsquerschnittsfläche für den Eintritt des Dampfs- und/oder des Gases durch den Spalt in das jeweilige Innere der komplementären Hälften des Luer-Verbinders definiert, die betragsmäßig mindestens der engsten Strömungsquerschnittsfläche des ersten Verbindungselements oder des zweiten Verbindungselements entspricht. Bei einem Luer-Lock-Konnektor kann die Strömungsquerschnittsfläche für den Eintritt des Dampfs- und/oder des Gases durch den Spalt in das jeweilige Innere der komplementären Hälften des Luer-Verbinders beispielsweise mindestens so groß sein wie die Strömungsquerschnittsfläche des Lumens der männlichen Hälfte des Luer-Verbinders.

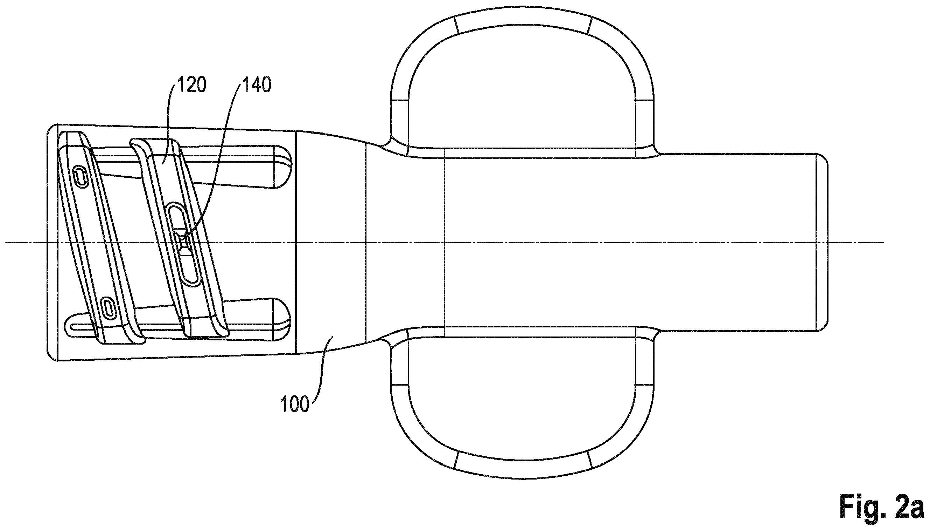

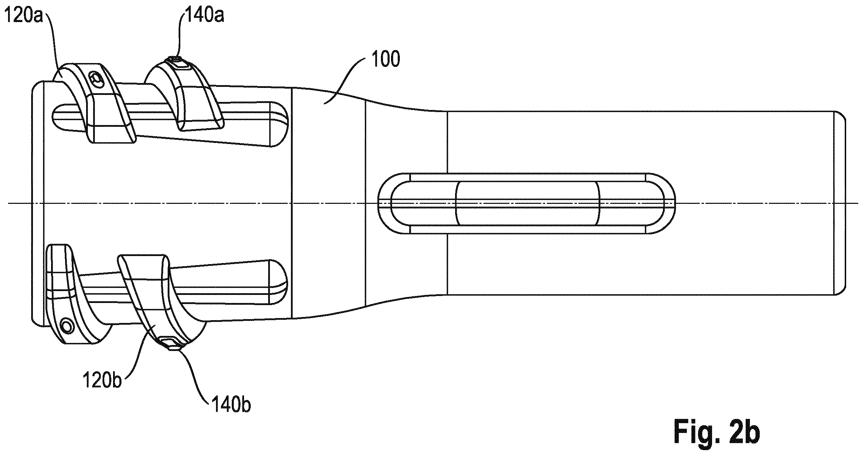

Die **Figuren 2a** und **2b** zeigen eine erste Ansicht einer alternativen Ausführungsform eines ersten Verbindungselements 100 und eine zweite Ansicht des ersten Verbindungselements 100, die gegenüber der ersten Ansicht um 90° um die Längsachse des ersten Verbindungselements 100 gedreht ist. Das Außengewinde 120 weist zumindest zwei erste Ausformungen 140a, 140b auf. Die ersten Ausformungen 140 sind als lokale Erhebungen, beispielsweise als Nocke oder Nase, auf der Gewindeflanke des Außengewindes 120 angeordnet und ragen über den Gewindedurchmesser des Außengewindes hinaus.

Der Schraubverbinder kann in anderen Ausführungsformen nur eine einzige erste Ausformung aufweisen.

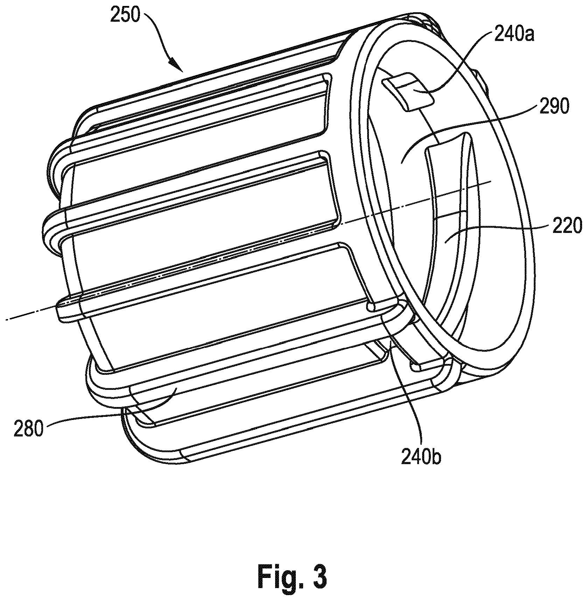

Die **Figur 3** zeigt eine perspektivische Ansicht des zweiten Verbindungselements 200, das als Abdichtkappe 250 ausgeführt ist. Die Abdichtkappe 250 ist an ihrem äußeren Umfang und über deren gesamte Länge mittels in axialer Richtung der Abdichtkappe verlaufender Versteifungsrippen 280 versteift, die außerdem die Griffigkeit beim manuellen Verschrauben verbessern. In Figur 3 ist ein Teil des Innengewindes 220 sichtbar und es sind gegenüberliegende zweite Ausformungen 240a und 240 b sichtbar, die als lokale Durchgangsöffnungen im Gewindegrund 290 ausgeführt sind.

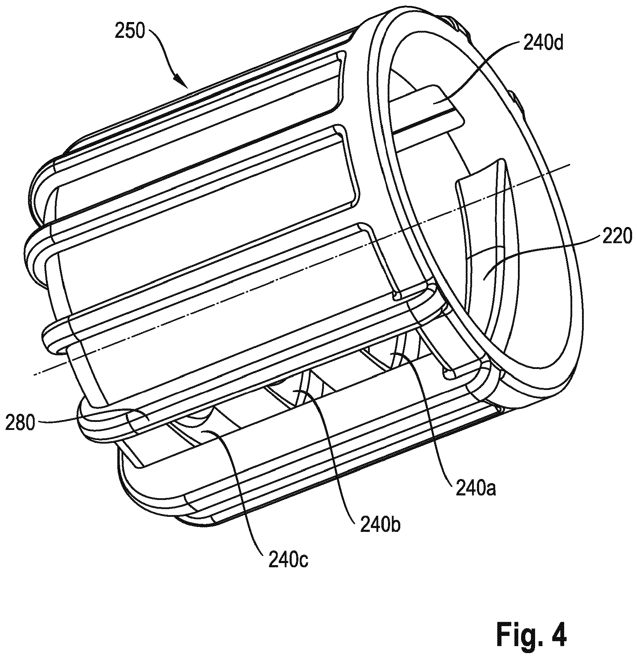

Die **Figur 4** zeigt eine alternative Ausführungsform des zweiten Verbindungselements aus Figur 3, wobei als einziger Unterschied zu dem Ausführungsbeispiel aus Figur 3 mehrere zweite Ausformungen in axialer Richtung der Abdichtkappe 250 vorgesehen sind, die sich als mehrere Durchgangsöffnungen 240a, 240b, 240c und 240d im Gewindegrund zwischen zwei benachbarten Versteifungsrippen 280 erstrecken. Diese Ausführungsform ermöglicht in Abhängigkeit von der Einschraubtiefe des Gewindes mehrere separate Rastpositionen.

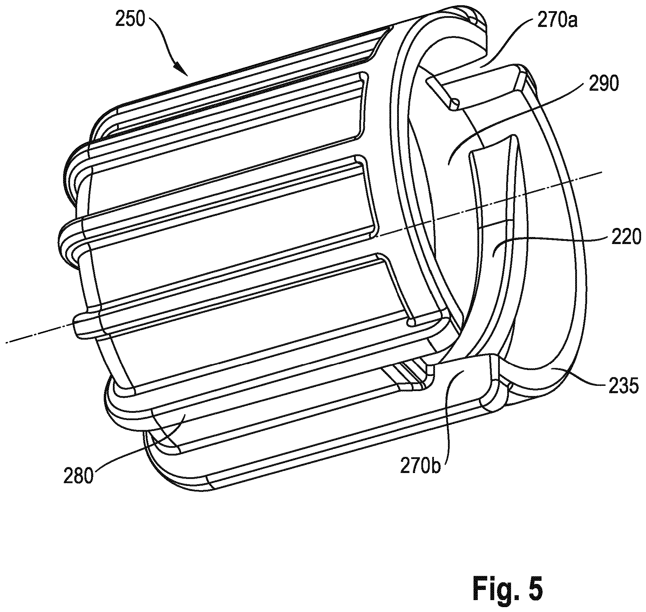

Die **Figur 5** zeigt eine weitere alternative Ausführungsform des zweiten Verbindungselements aus Figur 3, wobei als einziger Unterschied zu dem Ausführungsbeispiel aus Figur 3 die zweiten Ausformungen als radiale Nuten 270a und 270b in der offenen axialen Stirnseite der Abdichtkappe 250 ausgeführt sind und wobei die radialen Nuten 270a und 270b in Richtung der offenen axialen Stirnseite der Mutter 230 offen sind. Diese Ausführungsform ist besonders vorteilhaft hinsichtlich der Ausformung beim Spitzgießen.

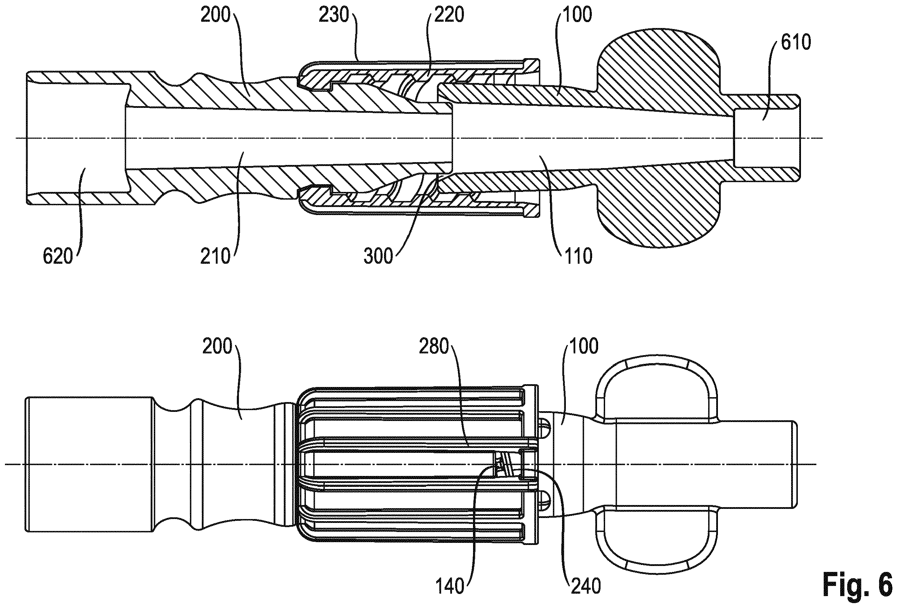

Die **Figur 6** zeigt in der oberen Abbildung einen Schnitt und in der unteren Abbildung eine Ansicht einer alternativen Ausführungsform des zweiten Verbindungselements 200 in Verbindung mit der Ausführungsform des ersten Verbindungselements 100 aus den Figuren 2a und 2b, wobei das zweite Verbindungselement 200 ein zweites Lumen 210 und eine Mutter 230 aufweist, die als Überwurfmutter ausgeführt ist. Die Überwurfmutter ist am zweiten Verbindungselement 200 drehbar gelagert, so dass eine Verschraubung der Überwurfmutter mit dem ersten Verbindungselement 100 möglich ist, ohne dass sich das gesamte zweite Verbindungselement 200 zusammen mit der Überwurfmutter mitdrehen muss. Das erste Verbindungselement 100 weist an seinem freien Ende eine erste Schlauchaufnahme 610 auf und das zweite Verbindungselement 200 weist an seinem freien Ende eine zweite Schlauchaufnahme 620 auf, die jeweils zum Einkleben von Schlauchabschnitten (nicht dargestellt) vorgesehen sind. Das erste Lumen ist als weibliche Hälfte eines Luer-Konnektors ausgebildet und das zweite Lumen ist als männliche Hälfte des Luer-Konnektors ausgebildet.

Die Schraubverbinder aus den Figuren 1 bis 6 können an medizinischen Schlauchsystemen eingesetzt werden und sind dafür vorgesehen.

Beispielsweise können die Schraubverbinder entsprechend Figuren 1 bis 6 an Schlauchsätzen für die extrakorporale Blutbehandlung eingesetzt werden. Blutschlauchsysteme für die extrakorporale Blutbehandlung weisen bei Anwendung eines Ein-Nadel-Zugangs zumindest einen Patientenschlauch oder bei Anwendung eines Zwei-Nadel-Zugangs einen arteriellen Patientenschlauch und einen venösen Patientenschlauch auf. Das jeweilige patientenseitige Ende eines jeden Patientenschlauchs kann jeweils mit einem erfindungsgemäßen Schraubverbinder oder einer Hälfte eines erfindungsgemäßen Schraubverbinders ausgestattet werden.

Manche extrakorporale Blutschlauchsätze weisen zumindest eine Zugabeleitung für die Zugabe eines Medikaments in den extrakorporalen Blutkreislauf auf, beispielsweise eine Heparinleitung und/oder eine Citratleitung und/oder eine Calciumleitung. Das freie Ende der Zugabeleitung kann mit einem Schraubverbinder gemäß den Figuren 1 bis 6 ausgestattet werden.

Es ist auch möglich eine Probenahmestelle an einem medizinischen Schlauchsystem mit einem Schraubverbinder gemäß den Figuren 1 bis 6 auszustatten.

In bestimmten Ausführungsformen eines extrakorporalen Blutkreislaufs ist das dem patientenseitige Ende des arteriellen Patientenschlauchs gegenüberliegende Ende des arteriellen Patientenschlauchs und das dem patientenseitige Ende des venösen Patientenschlauchs gegenüberliegende Ende des venösen Patientenschlauchs mit einer Blutkassette für die extrakorporale Blutbehandlung verbunden. In manchen dieser Ausführungsformen eines extrakorporalen Blutschlauchsatzes mit Blutkassette werden die patientenseitigen Enden des arteriellen Patientenschlauchs und des venösen Patientenschlauchs bereits bei der Produktion des extrakorporalen Blutschlauchsatzes mit zwei freien Anschlüssen eines T-Verbinders verbunden, wobei der T-Verbinder zum Befüllen und Spülen des Blutschlauchsatzes mit Primingflüssigkeit vor Beginn der extrakorporalen Blutbehandlung vorgesehen ist. Bei solchen Ausführungsformen können zumindest zwei freie Anschlüsse des T-Verbinders und die patientenseitigen Enden der beiden Patientenschläuche mit den komplementären Hälften eines Schraubverbinders gemäß den Figuren 1 bis 6 ausgestattet werden. Die patientenseitigen Enden des arteriellen Patientenschlauchs und des venösen Patientenschlauchs werden dann bereits bei der Produktion des extrakorporalen Blutschlauchsatzes in halboffener Sterilisationsstellung mit zwei freien Anschlüssen eines T-Verbinders verbunden, so dass bei der Sterilisation des extrakorporalen Blutschlauchsatzes Sterilisationsgas und/oder Sterilisationsdampf durch den Spalt des Schraubverbinders eindringen kann. Der dritte Anschluss des T-Verbinders kann mittels einer konventionellen Abdichtkappe abgedichtet sein oder aber auch mit einem Schraubverbinder entsprechend der vorliegenden Erfindung ausgestattet sein. Vor der Sterilisation wird der extrakorporale Blutschlauchsatz in einer Sterilverpackung verpackt. Vor der Anwendung des extrakorporalen Blutschlauchsatzes wird die Sterilverpackung geöffnet und der extrakorporalen Blutschlauchsatzes entnommen. In diesem Zustand befinden sich die erfindungsgemäßen Schraubverbinder noch in der halboffenen Sterilisationsstellung. Die beiden Schraubverbinder am T-Verbinder werden vom Anwender in der Klinik manuell dichtend zugedreht und der freie dritte Anschluss des Spülsteckers wird mit einer Quelle für Primingflüssigkeit verbunden. Nach dem Primen und Spülen des extrakorporalen Blutkreislaufs werden die patientenseitigen Enden der beiden Patientenschläuche vom T-Verbinder abgeschraubt und stehen für die Verbindung mit den Zugangsnadeln zur Verfügung, beispielsweise mit einer arteriellen Dialysenadel und einer venösen Dialysenadel.

Auch nicht gezeigte spezielle Ausführungsformen des zweiten Verbindungselements, beispielsweise die männliche Hälfte eines Luer-Lock-Verbinders mit einstückig starr verbundener Mutter, wie sie beispielsweise ohne die zweite Ausformung von Luer-Lock-Spritzen bekannt sind, sind im Einklang mit der Lehre der vorliegenden Erfindung. Auch solche spezielle Ausführungsformen des zweiten Verbindungselements können durch Spritzgießen mit einer zweiten Ausformung gefertigt werden. Solche Ausführungsformen können beispielsweise Teil eines Behälters, einer Tropfkammer oder einer medizinischen Flüssigkeitskassette, insbesondere einer Dialysekassette für die extrakorporale Blutbehandlung sein und ermöglichen den direkten Anschluss eines Schlauchabschnitts mit einem ersten Verbindungselement mit einer ersten Ausformung in der halboffenen Sterilisierstellung.

Bei einer automatisierten Fertigung medizinischer Schlauchsysteme, insbesondere bei der automatisierten Fertigung von Blutschlauchsätzen für die extrakorporale Blutbehandlung, ist die Verwendung eines Schraubverbinders nach der Lehre der vorliegenden Erfindung aufgrund der montagegerechte Konstruktion der Einzelteile für die Montage durch Roboter besonders vorteilhaft.

### Bezugszeichenliste

**Tabelle 1**

| **Bezugszeichen** | **Bezeichnung** |
|---|---|
| 100 | Erstes Verbindungselement |
| 110 | Erstes Lumen |
| 120, 120a, 120b | Außengewinde |
| 130 | Gewindegrund |
| 140, 140a, 140b | Erste Ausformung |
| 200 | Zweites Verbindungselement |
| 210 | Zweites Lumen |
| 220 | Innengewinde |
| 230 | Mutter |
| 235 | Stirnseite |
| 240, 240a, 240b, 240c, 240d | Zweite Ausformung |
| 250 | Abdichtkappe |
| 260 | Abdichtstruktur |
| 270, 270a, 270b | radiale Nut |
| 280 | Versteifungsrippe |
| 290 | Gewindegrund |
| 300 | Spalt |
| 400 | Drei-Wege-Verbinder |
| 500 | Medizinischer Schlauchsatz |
| 510 | Arterielle Patientenleitung |
| 511 | patientenseitiges Ende der arterielle Patientenleitung |
| 520 | Venöse Patientenleitung |
| 521 | patientenseitiges Ende der venösen Patientenleitung |
| 610 | erste Schlauchaufnahme |
| 620 | zweite Schlauchaufnahme |

## Patentansprüche

1. Schraubverbinder für ein medizinisches Schlauchsystem mit einem ersten Verbindungselement (100) mit einem ersten Lumen (110) und
einem Außengewinde (120) und einem zweiten Verbindungselement (200) mit einer elastisch verformbaren Mutter (230) mit einem Innengewinde (220),
wobei das Innengewinde (220) und das Außengewinde (120) konfiguriert sind um miteinander verschraubt zu werden wobei
das Außengewinde (120) eine erste Ausformung (140) aufweist und die Mutter (230) eine zweite Ausformung (240) aufweist, wobei die zweite Ausformung (240) eine Aussparung im Gewindegrund der Mutter (230) ist und
die Mutter (230) konfiguriert ist zur elastischen Verformung durch die erste Ausformung (140) in einem vorgegebenen ersten Drehwinkelbereich, ohne dass die erste Ausformung (140) in die Aussparung im Gewindegrund der Mutter (230) einrastet und
das Innengewinde (220) und das Außengewinde (120) und die erste Ausformung (140) und die Aussparung im Gewindegrund der Mutter (230) konfiguriert sind zum Einrasten der ersten Ausformung (140) in die Aussparung im Gewindegrund der Mutter (230), wenn das Innengewinde (220) und das Außengewinde (120) in einem vorgegebenen zweiten Drehwinkelbereich verschraubt sind,
**dadurch gekennzeichnet, dass**
das erste Verbindungselement (100) und das zweite Verbindungselement (200) jeweils konfiguriert sind um beim Verschrauben des Innengewindes (220) mit dem Außengewinde (120) in dem zweiten Drehwinkelbereich gasdurchlässig zu sein zwischen einer äußeren Umgebung des Schraubverbinders und einem zumindest durch das erste Lumen (110) gebildeten Inneren des Schraubverbinders und
das erste Verbindungselement (100) und das zweite Verbindungselement (200) jeweils konfiguriert sind um beim Verschrauben des Innengewindes (220) mit dem Außengewinde (120) in einem weiteren Drehwinkelbereich gas- und/ oder flüssigkeitsdicht zwischen der äußeren Umgebung des Schraubverbinders und dem Inneren des Schraubverbinders abzudichten.

2. Schraubverbinder gemäß Anspruch 1, wobei die erste Ausformung (140) eine lokale Erhebung auf der Gewindeflanke des Außengewindes (120), insbesondere eine Nase oder eine Nocke auf dem Außendurchmesser des Außengewindes (120), ist.

3. Schraubverbinder gemäß einem der Ansprüche 1 bis 2, wobei die Aussparung im Gewindegrund der Mutter (230) zumindest eine Durchgangsöffnung zwischen dem Inneren der Mutter (230) und einer äußeren Umgebung der Mutter (230) umfasst.

4. Schraubverbinder gemäß Anspruch 3, wobei die zumindest eine Durchgangsöffnung eine radiale Nut in einer axialen Stirnseite der Mutter (230) umfasst und wobei die radiale Nut in Richtung der axialen Stirnseite der Mutter (230) offen ist.

5. Schraubverbinder gemäß einem der Ansprüche 3 bis 4, wobei die Mutter (230) an ihrem äußeren Umfang zumindest eine in axialer Richtung der Mutter (230) angeordnete Versteifungsrippe aufweist und sich die mindestens eine Durchgangsöffnung zumindest teilweise entlang der Versteifungsrippe erstreckt.

6. Schraubverbinder gemäß einem der Ansprüche 1 bis 5, wobei das erste Verbindungselement (100) und das zweite Verbindungselement (200) jeweils als komplementäre Hälfte eines gemeinsamen Luer-Lock Verbinders ausgeführt sind.

7. Schraubverbinder gemäß Anspruch 1 bis 6, wobei das zweite Verbindungselement (200) eine Abdichtkappe (250) mit einer Abdichtstruktur (260) aufweist.

8. Schraubverbinder gemäß Anspruch 7, wobei das erste Lumen (110) als weibliche Hälfte des Luer-Konnektors ausgebildet ist und die Abdichtstruktur (260) als männliche Hälfte des Luer-Konnektors ausgebildet ist.

9. Schraubverbinder gemäß einem der Ansprüche 1 bis 6, wobei das zweite Verbindungselement (200) ein zweites Lumen (210) aufweist und die Mutter (230) als Überwurfmutter konfiguriert ist.

10. Schraubverbinder gemäß Anspruch 9, wobei das erste Lumen (110) als weibliche Hälfte des Luer-Konnektors ausgebildet ist und das zweite Lumen (210) als männliche Hälfte des Luer-Konnektors ausgebildet ist.

11. Medizinisches Schlauchsystem mit einem Schraubverbinder gemäß einem der Ansprüche 1 bis 10, wobei das medizinische Schlauchsystem einen Blutschlauchsatz für die extrakorporale Blutbehandlung mit einem arteriellen Patientenschlauch und einem venösen Patientenschlauch aufweist und das patientenseitige Ende des arteriellen Patientenschlauchs und das patientenseitige Ende des venösen Patientenschlauchs jeweils als das zweite Verbindungselement (200) ausgebildet ist.

12. Medizinisches Schlauchsystem gemäß Anspruch 11, wobei das medizinische Schlauchsystem einen Drei-Wege-Verbinder (400) mit drei verbundenen Lumen aufweist, wobei mindestens zwei der drei verbundenen Lumen jeweils als das erste Verbindungselement (100) ausgestaltet sind.

13. Medizinisches Schlauchsystem gemäß Anspruch 12 konfiguriert zum Kurzschließen des patientenseitigen Endes des arteriellen Patientenschlauchs und des patientenseitige Ende des venösen Patientenschlauchs mit dem Drei-Wege-Verbinder (400).

14. Medizinisches Schlauchsystem gemäß einem der Ansprüche 11 bis 13, wobei das dem patientenseitigen Ende des arteriellen Patientenschlauchs gegenüber liegende Ende des arteriellen Patientenschlauchs und das dem patientenseitigen Ende des venösen Patientenschlauchs gegenüber liegende Ende des venösen Patientenschlauchs mit einer Blutkassette für die extrakorporale Blutbehandlung verbunden sind.

15. Medizinisches Schlauchsystem gemäß einem der Ansprüche 11 bis 14, wobei das medizinische Schlauchsystem mindestens eine Zugabestelle für medizinische Flüssigkeiten aufweist, wobei die Zugabestelle als das erste Verbindungselement (100) zum Verbinden mit einer als das zweite Verbindungselement (200) ausgestalteten Abdichtkappe (250) ausgebildet ist.

16. Verwenden eines Schraubverbinders gemäß einem der Ansprüche 1 bis 10 zum Gassterilisieren und/oder Dampfsterilisieren eines medizinischen Schlauchsystems gemäß einem der Ansprüche 11 bis 15.

## Claims

1. A screw connector for a medical tubing system having a first connecting element (100) with a first lumen (110) and an outside thread (120) and a second connecting element (200) with an elastically deformable nut (230) having an inside thread (220),
wherein the inside thread (220) and the outside thread (120) are configured to be screwed together, wherein
the outside thread (120) has a first formation (140) and the nut (230) has a second formation (240), wherein the second formation (240) is a recess in the base of the thread on the nut (230), and
the nut (230) is configured for elastic deformation by the first formation (140) in a predetermined first angle-of-rotation range, without the first formation (140) engaging in the recess in the base of the thread of the nut (230), and
the inside thread (220) and the outside thread (120) and the first formation (140) and the base of the thread on the nut (230) are configured for engagement of the first formation (140) in the recess in the base of the thread on the nut (230), when the inside thread (220) and the outside thread (120) are screwed in a predetermined second angle-of-rotation range,
**characterized in that**
the first connecting element (100) and the second connecting element (200) are each configured to be gas-permeable between the outside surroundings of the screw connector and an interior of the screw connector formed at least by the first lumen (110) when the inside thread (220) is screwed together with the outside thread (120) in the second angle-of-rotation range, and
the first connecting element (100) and the second connecting element (200) are each configured to form an air-tight and/or fluid-tight seal between the outer surroundings of the screw connector and the interior of the screw connector when the inside thread (220) is screwed together with the outside thread (120) in another angle-of-rotation range.

2. The screw connector according to claim 1, wherein the first formation (140) is a local elevation on the thread flank of the outside thread (120), in particular a nub or a cam on the outside diameter of the outside thread (120).

3. The screw connector according to any one of claims 1 to 2, wherein the recess in the base of the thread of the nut (230) comprises at least a through-opening between the interior of the nut (230) and an outside circumference of the nut (230).

4. The screw connector according to claim 3, wherein the at least one through-opening is a radial groove in an axial end face of the nut (230), and wherein the radial groove is open in the direction of the axial end face of the nut (230).

5. The screw connector according to any one of claims 3 to 4, wherein the nut (230) has at least one reinforcing rib arranged in the axial direction of the nut (230) on its outside circumference, and the at least one through-opening extends at least partially along the reinforcing rib.

6. The screw connector according to any one of claims 1 to 5, wherein the first connecting element (100) and the second connecting element (200) are each designed as complementary halves of a joint Luer-Lock connector.

7. The screw connector according to claims 1 to 6, wherein the second connecting element (200) has a sealing cap (250) with a sealing structure (260).

8. The screw connector according to claim 7, wherein the first lumen (110) is designed as the female half of the Luer connector, and the sealing structure (260) is designed as the male half of the Luer connector.

9. The screw connector according to any one of claims 1 to 6, wherein the second connecting element (200) has a second lumen (210), and the nut (230) is configured as a union nut.

10. The screw connector according to claim 9, wherein the first lumen (110) is designed as the female half of the Luer connector, and the second lumen (210) is designed as the male half of the Luer connector.

11. The medical tubing system having a screw connector according to any one of claims 1 to 10, wherein the medical tubing system has a blood tubing set for the extracorporeal blood treatment with an arterial patient tube and a venous patient tube, and the patient's end of the venous patent tube is designed as the second connecting element (200).

12. The medical tubing system according to claim 11, wherein the medical tubing system has a three-way connector (400) with three connected lumens, wherein at least two of the three connected lumens are each designed as the first connecting element (100).

13. The medical tubing system according to claim 12, configured for short-circuiting the patient's end of the patient's arterial tube and the patient's end of the patient's venous tube with the three-way connector (400) .

14. The medical tubing system according to any one of claims 11 to 13, wherein the end of the patient's arterial tube opposite the patient's end of the patient's arterial tube and the end of the patient's venous tube opposite the patient's end of the patient's venous tube are connected to a blood cassette for the extracorporeal blood treatment.

15. The medical tubing system according to any one of claims 11 to 14, wherein the medical tubing system has at least one addition point for medical fluids, wherein the addition port is designed as the first connecting element (100) for connecting to a sealing cap (250) designed as the second connecting element (200) .

16. Use of a screw connector according to any one of claims 1 to 10, for gas sterilization and/or steam sterilization of a medical tubing system according to any one of claims 11 to 15.

## Revendications

1. Connecteur à vis pour système tubulaire médical, comportant un premier élément de connexion (100) doté d'un premier lumen (110) et d'un filetage extérieur (120) et un second élément de connexion (200) doté d'un écrou déformable élastiquement (230) comportant un filetage intérieur (220),
le filetage intérieur (220) et le filetage extérieur (120) étant conformés pour être vissés ensemble,
le filetage extérieur (120) présentant un premier façonnage (140) et l'écrou (230) un second façonnage (240), le second façonnage (240) étant un évidement dans la base filetée de l'écrou (230) et
l'écrou (230) étant conformé avec un tarif 10 pour une déformation élastique par le premier façonnage (140) dans une première plage prédéfinie d'angle de rotation sans que le premier façonnage (140) s'enclenche dans le façonnage de la base filetée de l'écrou (230) est
le filetage intérieur (220) et le filetage extérieur (120) et le premier façonnage (140) et l'évidement de la base filetée de l'écrou (230) étant conformés pour un enclenchement du premier façonnage (140) dans l'évidement de la base filetée de l'écrou (230) lorsque le filetage intérieur (220) et le filetage extérieur (120) sont vissés dans une seconde plage prédéfinie d'angle de rotation,
**caractérisé en ce que**
le premier élément de connexion (100) et le second élément de connexion (200) sont respectivement conformés pour, en cas de vissage du filetage intérieur (220) avec le filetage extérieur (120) dans la seconde plage d'angle de rotation, être perméables au gaz entre un environnement extérieur du connecteur à vis et un intérieur constitué du moins par le premier lumen (110) du connecteur à vis et que
le premier élément de connexion (100) et le second élément de connexion (200) sont respectivement conformés pour, en cas de vissage du filetage intérieur (220) avec le filetage extérieur (120) dans une autre plage d'angle de rotation, assurer une étanchéité au gaz et/ou au liquide entre un environnement extérieur du connecteur à vis et l'intérieur du connecteur à vis.

2. Connecteur à vis selon la revendication 1, dans lequel le premier façonnage (140) est une proéminence locale sur le flanc fileté du filetage extérieur (120), en particulier un bec ou une came sur le diamètre extérieur du filetage extérieur (120).

3. Connecteur à vis selon une des revendications 1 à 2, dans lequel l'évidement de la base de l'écrou (230) comprend au moins une ouverture de passage entre l'intérieur de l'écrou (230) et un environnement extérieur de l'écrou (230).

4. Connecteur à vis selon la revendication 3, dans lequel l'au moins une ouverture de passage comprend une rainure radiale dans une face avant axiale de l'écrou (230) et la rainure radiale est ouverte en direction de la face avant axiale de l'écrou (230).

5. Connecteur à vis selon une des revendications 3 à 4, dans lequel l'écrou (230) présente, au niveau de sa circonférence extérieure, au moins une nervure de renfort disposée dans un sens axial de l'écrou (230) et l'au moins une ouverture de passage s'étend du moins partiellement le long de la nervure de renfort.

6. Connecteur à vis selon une des revendications 1 à 5, dans lequel le premier élément de connexion (100) et le second élément de connexion (200) sont respectivement réalisés sous forme de moitié complémentaire d'un connecteur luer lock commun.

7. Connecteur à vis selon une des revendications 1 à 6, dans lequel le second élément de connexion (200) présente un capot d'étanchéité (250) doté d'une structure d'étanchéité (260).

8. Connecteur à vis selon la revendication 7, dans lequel le premier lumen (110) est réalisé sous forme d'une moitié femelle du connecteur luer lock et la structure d'étanchéité (260) sous forme d'une moitié mâle du connecteur luer lock.

9. Connecteur à vis selon une des revendications 1 à 6, dans lequel le second élément de connexion (200) présente un second lumen (210) et l'écrou (230) est réalisé sous forme d'un écrou à chapeau.

10. Connecteur à vis selon la revendication 9, dans lequel le premier lumen (110) est réalisé sous forme d'une moitié femelle du connecteur luer lock et le second lumen (210) est réalisé sous forme d'une moitié mâle du connecteur luer lock.

11. Système tubulaire médical comportant un connecteur à vis selon une des revendications 1 à 10, ce système tubulaire médical présentant un jeu de tuyaux à sang pour le traitement sanguin extracorporel, comportant un tuyau artériel pour patient et un tuyau veineux pour patient et l'extrémité située du côté du patient du tuyau artériel pour patient, et l'extrémité située du côté du patient du tuyau veineux pour patient étant respectivement réalisée sous forme du second élément de connexion (200).

12. Système tubulaire médical selon la revendication 11, ce système tubulaire médical présentant un connecteur à trois voies (400) comportant trois lumens connectés, au moins deux des trois lumens connectés étant réalisés sous forme du premier élément de connexion (100) .

13. Système tubulaire médical selon la revendication 12, conçu pour court-circuiter l'extrémité située du côté du patient du tuyau artériel pour patient et l'extrémité située du côté du patient du tuyau veineux pour patient avec le connecteur à trois voies (400).

14. Système tubulaire médical selon une des revendications 11 à 13, dans lequel l'extrémité opposée à l'extrémité située du côté du patient du tuyau artériel pour patient et l'extrémité opposée à l'extrémité située du côté du patient du tuyau veineux pour patient du tuyau veineux pour patient sont connectées à une cassette à sang pour le traitement sanguin extracorporel.

15. Système tubulaire médical selon une des revendications 11 à 14, dans lequel le système tubulaire médical présente au moins un point d'administration pour des liquides médicaux, le point d'administration étant réalisé sous forme du premier élément de connexion (100) destiné à être connecté à un capot d'étanchéité (250) réalisé sous forme du second élément de connexion (200).

16. Utilisation d'un connecteur à vis selon une des revendications 1 à 10 pour la stérilisation au gaz et/ou la stérilisation à la vapeur d'un système tubulaire médical selon une des revendications 11 à 15.
